# EUROPÄISCHE PATENTSCHRIFT

(11) **EP 1 506 776 B1**
(45) Veröffentlichungstag und Bekanntmachung des Hinweises auf die Patenterteilung: **03.03.2010**
(21) Anmeldenummer: 04018956.5
(22) Anmeldetag: 10.08.2004
(51) Int. Cl.: A61K 31/165, A61K 38/00, A61K 38/05, A61P 25/16

(54) **Verwendung der Inhibitoren von Enzymen mit Aktivitäten der Aminopeptidase N und/oder der Dipeptidylpeptidase IV und pharmazeutischen Zubereitungen daraus zur Therapie und Prävention von chronischen neurodegenerativen Erkrankungen**
Use of enzyme inhibitors of the dipeptidylpeptidase IV and/or of the aminopeptidase N and/or pharmaceutical preparations thereof for the prevention or therapy of neurodegenerative diseases
Utilisation des inhibiteurs enzymatiques de la dipeptidylpeptidase IV et/ou de l'aminopeptidase n, et préparations pharmaceutiques les contenant pour la prévention ou le traitement des maldies neurodégéneratives

(30) Priorität: 12.08.2003 DE 10337074
(43) Veröffentlichungstag der Anmeldung: 16.02.2005
(73) Patentinhaber: KeyNeurotek Pharmaceuticals AG, 39120 Magdeburg (DE)
(72) Erfinder: Striggow, Frank, Dr., 39120 Magdeburg (DE); Röhnert, Peter, Dr., 39120 Magdeburg (DE); Mack, Till, Dr., 39120 Magdeburg (DE)
(74) Vertreter: Koepe, Gerd L.

(56) Entgegenhaltungen:
- WO-A-01/34594
- WO-A-93/07872
- WO-A-02/053169
- WU YONG-QIAN ET AL: "Neuroprotective effects of inhibitors of dipeptidyl peptidase-IV in vitro and in vivo." DIPEPTIDYL AMINOPEPTIDASES IN HEALTH AND DISEASE KLUWER ACADEMIC/PLENUM PUBLISHERS, 233 SPRING STREET, NEW YORK, NY, 10013-1578, USA SERIES : ADVANCES IN EXPERIMENTAL MEDICINE AND BIOLOGY (ISSN 0065-2598), 2003, Seiten 351-355, XP009037230 & INTERNATIONAL CONFERENCE ON DIPEPTIDYL AMINOPEPTIDASES; BERLIN, GERMANY; SEPTEMBER 26-28, 2002 ISSN: 0-306-47717-3
- DATABASE MEDLINE [Online] US NATIONAL LIBRARY OF MEDICINE (NLM), BETHESDA, MD, US; Januar 1992 (1992-01), KRYZHANOVSKII G N ET AL: "[Effects of intranasally administered substance P in parkinsonian syndrome]" XP002298708 Database accession no. NLM1382692 & BIULLETEN' EKSPERIMENTAL'NOI BIOLOGII I MEDITSINY. JAN 1992, Bd. 113, Nr. 1, Januar 1992 (1992-01), Seiten 16-19, ISSN: 0365-9615
- GABRILOVAC JELKA ET AL: "Expression of CD13/aminopeptidase N and CD10/neutral endopeptidase on cultured human keratinocytes." IMMUNOLOGY LETTERS, Bd. 91, Nr. 1, 30. Januar 2004 (2004-01-30), Seiten 39-47, XP002298707 ISSN: 0165-2478
- AOYAGI TAKAAKI ET AL: "Enzymatic changes in cerebrospinal fluid of patients with Alzheimer-type dementia" JOURNAL OF CLINICAL BIOCHEMISTRY AND NUTRITION, Bd. 14, Nr. 2, 1993, Seiten 133-139, XP009037233 ISSN: 0912-0009
- LENDECKEL UWE ET AL: "Synergistic action of DPIV and APN in the regulation of T cell function." DIPEPTIDYL AMINOPEPTIDASES IN HEALTH AND DISEASE KLUWER ACADEMIC/PLENUM PUBLISHERS, 233 SPRING STREET, NEW YORK, NY, 10013-1578, USA SERIES : ADVANCES IN EXPERIMENTAL MEDICINE AND BIOLOGY (ISSN 0065-2598), 2003, Seiten 123-131, XP001183828 & INTERNATIONAL CONFERENCE ON DIPEPTIDYL AMINOPEPTIDASES; BERLIN, GERMANY; SEPTEMBER 26-28, 2002 ISSN: 0-306-47717-3
- DATABASE WPI Section Ch, Week 200456 Derwent Publications Ltd., London, GB; Class B05, AN 2004-580657 XP002298709 & WO 2004/064866 A1 (HAMARI CHEM LTD) 5. August 2004 (2004-08-05)

## Beschreibung

Die Erfindung beschreibt die Verzögerung der progressiven Alzheimerspezifischen Neurodegeneration und damit Verbesserungen von pathophysiologischen und kognitiven Parametern durch die Wirkung von Inhibitoren der Aminopeptidase N (APN, EC 3.4.11.2, CD13) und der Dipeptidylpeptidase IV (DP IV, EC 3.4.14.5, CD26) im Ergebnis der, simultanen oder zeitlich unmittelbar aufeinanderfolgenden Applikation von jeweils spezifischen Inhibitoren dieser Enzyme oder von Inhibitoren von Enzymen gleicher Substratspezifität (APN- und DP IV-analoge Enzymaktivität) auf der Basis von Aminosäurederivaten, Peptiden oder Peptidderivaten.

Es gibt eine Reihe von altersbedingten Demenzerkrankungen, von denen die Alzheimer'sche Demenz eine besonders wichtige Rolle spielt. Im Jahr 2001 waren in den USA, Japan und Westeuropa etwa 6,5 Millionen Menschen davon betroffen. Aufgrund der alternden Bevölkerung ist in den "entwickelten" Ländern mit einer starken Zunahme der Erkrankungen zu rechnen.

Zur Entstehung der Alzheimer'schen Demenz gibt es verschiedene Hypothesen. Die meisten beruhen auf Befunden, die an Patienten mit familiär vererbter Alzheimer'scher Demenz erhoben worden sind. Es wird allgemein angenommen, dass Störungen bei der Prozessierung von zwei Proteinen, dem Amyloid Precursor Protein und dem tau-Protein, zur Ansammlung von charakteristisehen Proteinablagerungen, zu Defiziten in der Neurotransmission und schließlich zum Untergang von Nervenzellen führen.

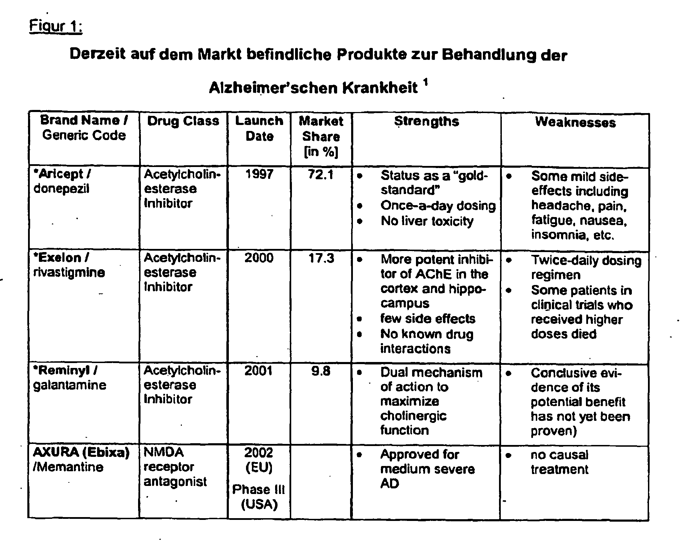

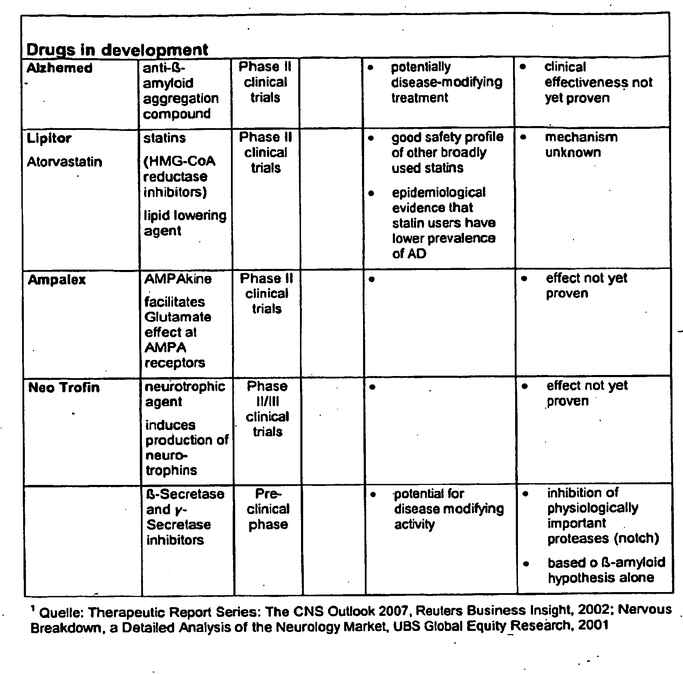

Zu Behandlung sind bisher fast ausschließlich Therapeutika vom Typ der Acetylcholinesterase-Inhibitoren (AChEls) zugelassen. Seit August 2002 ist in Deutschland auch der Wirkstoff Memantine (AXURA), ein Antagonist des NMDA-Rezeptors, auf dem Markt (Therapeutic Report Series: The CNS Outlook to 2007, Reuters Business Insight, 2002, siehe Figur 1).

AchEls erhöhen die Konzentration des Neurotransmitters Acetylcholin und führen zu leichten Verbesserungen der kognitiven Leistungen und der Aktivitäten des täglichen Lebens (activities associated with daily living). Sie haben relativ geringe Nebenwirkungen (Magen-Darm) und sind wenig toxisch.

Durch Behandlung mit AchEls wird das Fortschreiten der Demenz etwas (0,5 bis 1 Jahr) verzögert; eine kausale Therapie ist jedoch nicht möglich. Zur Behandlung von fortgeschrittenen Krankheitsstadien ist nur Memantine zugelassen. Die Therapie mit diesem Wirkstoff beruht auf der Annahme, dass eine erhöhte Aktivität des NMDA-Rezeptors bei der Alzheimer-Pathologie beteiligt ist. Die Substanz hat ein gutes Sicherheitsprofil (unter dem Handelsnamen Akatinol seit Jahren bei "Hirnleistungsstörungen" verordnet). Über langfristige Effekte auf den Krankheitsverlauf ist noch nichts bekannt. Ein Einfluss auf die Grunderkrankung kann nicht erwartet werden.

In der frühen klinischen Prüfung befinden sich mehrere Wirkstoffe mit verschiedenen Wirkprinzipien (siehe Figur 1). Inwieweit diese Substanzen zu symptomatischen Verbesserungen oder Verlangsamung des Krankheitsverlaufs führen, bleibt abzuwarten.

Besonders hohe Erwartungen werden an die Hemmstoffe der β- oder γ-Secretase gestellt. Diese Substanzen hemmen die Bildung des β-Amyloids, des Hauptbestandteils der neuritischen Plaques. Aufgrund unspezifischer Proteasehemmung haben bisher getestete Wirkstoffe ein hohes Nebenwirkungspotential. Darüber hinaus wird von einigen Wissenschaftlern bezweifelt, dass die Bildung von β-Amyloid die alleinige Ursache für die Alzheimer-Pathologie ist, denn die β-Amyloid-Ablagerungen korrelieren nicht mit dem Schweregrad der Krankheit und bieten keine Erklärung für die Bildung von Tau-Bündeln (neurofibrilläre Tangles = NFT).

Die bisher zur Verfügung stehenden Arzneimittel erlauben keine effektive Behandlung der Alzheimer'schen Demenz; neue therapeutische Ansätze sind daher dringend erforderlich.

Peptidasen wie die Dipeptidylpetidase IV und die Aminopeptidase N oder ähnlich wirkende Enzyme sind für die Regulation bzw. Modulation von Wechselwirkungen zwischen Zellen besonders interessant, da sie zum Teil als Ektoenzyme in der Plasmamembran der Zellen lokalisiert sind, Interaktionen mit anderen extrazellulären Strukturen eingehen, peptiderge Botenstoffe durch enzymkatalysierte Hydrolyse aktivieren bzw. inaktivieren und dadurch wichtig für die Zell-Zell-Kommunikation sind [Yaron A, et al.: Proline-dependent structural and biological properties of peptides and proteins. Crit Rev Biochem Mol Biol 1993;28:31-81; Vanhoof G, et al.: Proline motifs in peptides and their biological processing. FASEB J 1995;9:736-744].

Es ist gezeigt worden, dass im Prozess der Aktivierung und klonalen Expansion von Immunzellen, insbesondere von T-Lymphozyten, membranständige Peptidasen wie DP IV oder APN eine Schlüsselrolle spielen [Fleischer B: CD26 a surface protease involved in T-cell activation. Immunology Today 1994; 15:180-184; Lendeckel U et al.: Role of alanyl aminopeptidase in growth and function of human T cells. International Journal of Molecular Medicine 1999; 4:17-27; Riemann D et al.: CD13 - not just a marker in leukemia typing. Immunology Today 1999; 20:83-88]. Verschiedene Funktionen Mitogenstimulierter mononukleärer Zellen (MNZ) oder angereicherter T-Lymphozyten wie DNA-Synthese, Produktion und Sekretion von immunstimulierenden Zytokinen (IL-2, IL-6, IL-12, IFN-γ) und Helferfunktionen für B-Zellen (IgG- und IgM-Synthese) können in Gegenwart von spezifischen Inhibitoren der DP IV oder der APN gehemmt werden [Schön E et al.: The dipeptidyl peptidase IV, a membrane enzyme involved in the proliferation of T lymphocytes. Biomed. Biochim. Acta 1985; 2: K9-K15; Schön E et al.: The role of dipeptidyl peptidase IV in human T lymphocyte activation. Inhibitors and antibodies against dipeptidyl peptidase IV suppress lymphocyte proliferation and immunoglobulin synthesis in vitro. Eur. J. Immunol. 1987; 17: 1821-1826; Reinhold D et al.: Inhibitors of dipeptidyl peptidase IV induce secretion of transforming growth factor β1 in PWM-stimulated PBMNC and T cells. Immunology 1997; 91: 354-360; Lendeckel U et al.: Induction of the membrane alanyl aminopeptidase gene and surface expression in human T-cells by mitogenic activation. Biochem. J. 1996; 319: 817-823; Kähne T et al.: Dipeptidyl peptidase IV: A cell surface peptidase involved in regulating T cell growth (Review). Int. J. Mol. Med. 1999; 4: 3-15; Lendeckel U et al.: Role of alanyl aminopeptidase in growth and function of human T cells (Review). Int. J. Mol. Med. 1999; 4: 17-27].

Es ist bereits bekannt, daß die Behandlung von Autoimmunerkrankungen und Transplantatabstoßung durch Hemmung der auf Immunzellen lokalisierten Dipeptidylpetidase IV mit Hilfe von synthetischen Inhibitoren möglich ist (z. B. EP-A 0 764151, WO095/29691, EP-A 0 731 789, EP-A 0 528 858).

Die Druckschrift WO 93/07872 beschreibt die Inhibierung von lysosomalen Enzymen u.a. mittels Actinonin und Bestatin sowie deren Verwendung zur Behandlung von neurodegenerativen Erkrankungen wie beispielsweise Morbus Alzheimer und Morbus Creutzfeldt-Jakob.

Die Druckschrift WO 02/053169 offenbart pharmazeutische Zubereitungen, die eine Kombination aus Inhibitoren der Dipeptidylpeptidase IV und Aminopeptidase N umfassen, sowie deren Verwendung zur Prävention und Therapie ischämieinduzierter Schädigungen im zentralen Nervensystem, vorzugsweise nach ischämischem oder hämorragischem Schlaganfall, nach Schädel/Hirn-Trauma, nach Herzstillstand, nach Herzinfarkt oder als Folge von herzchirurgischen Eingriffen.

Die Druckschrift WO 01/34594 beschreibt Inhibitoren der Dipeptidylpeptidase IV, die einen Prolin-Imitator umfassen, sowie deren Verwendung zur Behandlung von Erkrankungen des zentralen Nervensystems wie beispielsweise Schlaganfall, Ischämie, Morbus Parkinson, etc..

Das Dokument "Wu, Yong-Qian et al. "Neuroprotective effects of inhibitors of dipeptidyl peptidase-IV in vitro and in vivo" in "Dipeptidyl Aminopeptidases in Health and Disease", Kluwer Academic/Plenum Publishers, New York, 2003, Seiten 351 - 355° betrifft die neuroprotektive Wirkung bestimmter Dipeptidylpeptidase IV-Inhibitoren.

In dem Dokument "Database Medline US National Library of Medicine (NLM), Bethesda, MD, US, Januar 1992, Kryzhanovskii, G.N. et al. "Effects of intranasally administered substance P in parikinsonian syndrome"" wird die Wirkung der intranasalen Verabreichung von Substanz auf Morbus Parkinson beschrieben.

Die Druckschrift WO 2004/064866 offenbart die alleinige Verwendung von Bestatin zur Behandlung von Morbus Alzheimer und Demenz.

Der Erfindung liegt der überraschende Befund zugrunde, dass die gleichzeitige Wirkung von Inhibitoren der intraneuronalen Dipeptidylpeptidase IV / DP IV bzw. CD26 oder von Inhibitoren von Enzymen mit gleicher Substratspezifität (DP IV-analoger (Enzymaktivität) und von Inhibitoren der Aminopeptidase N / APN bzw. CD13 oder von Inhibitoren von Enzymen mit gleicher Substratspezifität (APN-analoger Enzymaktivität), die Bildung von neurofibrillären Tangles (NFT) verlangsamen kann.

Unsere Erfindung zeigt, dass zur Therapie und zur Prävention von insbesondere neurodegenerativen Erkrankungen mit Ausbildung neufibrillärer Tangles (NFT), d. h. von sogenannten Tauopathien, die gleichzeitige Applikation von Hemmstoffen der DP IV und der APN oder von Hemmstoffen von Enzymen gleicher Substratspezifität (APN- oder/und DP IV-analoger Enzymaktivität) bzw. entsprechender Zubereitungen und Darreichungsformen daraus geeignet sind.

Im einzelnen liegen der Erfindung die Befunde zugrunde, dass die Anzahl von neurofibrillären Tangles (NFT) im Hippocampus und anderen Hirnbereichen (z. B. Amygdala) durch die Gabe von Inhibitoren der Dipeptidylpeptide IV oder von Inhibitoren von Enzymen mit gleicher Substratspezifität (DP IV-analoger Enzymaktivität) und von Inhibitoren der Aminopeptidase N oder von Inhibitoren von Enzymen mit gleicher Substratspeztität (APN-analoger Enzymaktivität) signifikant reduziert wird. Besondere Indikationsbereiche für die erfindungsgemäße Verwendung sind menschliche Erkrankungen mit NFT, d. h. sogenannte Tauopathien. Dazu zählen insbesondere, ohne die Erfindung darauf zu beschränken, Morbus Alzheimer, die Pick'sche Erkrankung, die progressive supranukleäre Palsy, die kortikobasale Degeneration und die frontotemporale Demenz. Weitere neurodegenerative Erkrankungen, bei denen mißgefaltete Proteine in charakteristischen Läsionen akkumulieren und wesentlich zur Pathogenese und damit zum selektiven Untergang von Nervenzellen beitragen, sind Morbus Parkinson, Morbus Huntington und durch Prionen verursachte Erkrankungen (z. B. spongiforme Enzephalopathie.

Die Parkinson-Krankheit wird bislang, wie bereits für Morbus Alzheimer beschrieben, ausschließlich auf der Ebene der Symptome behandelt, d.h. praktisch häufig mit einer Kombination von Levodopa (Vorstufe des Transmitters der betroffenen Nervenzellen) und Dopamin-Agonisten. Bei derartiger Medikation treten häufig unerwünschte Nebenwirkungen in Form von Dyskinesien (Blockaden) auf. Darüber hinaus nutzt sich nach einigen Jahren der Effekt stark ab. Wie auch für Morbus Alzheimer gilt, daß bislang noch keine an der Pathogenese ansetzende, d.h. auf die Ursachen der Erkrankung einwirkende Therapieform existiert.

Der Körper reagiert auf verschiedenste Herauforderungen mit Inflammation, ein Vorgang, der gefährliche Agenzien beseitigen und ihre schädliche Wirkung beseitigen soll. Im Verlauf von neurodegenerativen Erkrankungen wird Inflammation vermutlich durch die abnormale Akkumulation missgefalteter Proteine und/oder Signale von pathologisch veränderten Neuronen ausgelöst. Veränderte Expression der an diesen inflammatorischen Prozessen beteiligten Faktoren kann die neurodegenerative Erkrankung sowohl befördern als auch ihr entgegenwirken (Wyss-Coray, T and Mucke, L: Inflammation in Neurodegenerative disease - a double -edged sword. Neuron 2002; 35:419-432). Da also Inflammation durchaus positive, neuroprotektive Aspekte hat, erscheint eine Lenkung bzw. gezielte Elimination der negativen Aspekte ein besserer therapeutischer Ansatz als derjenige, Inflammation grundsätzlich zu unterdrücken.

Durch eine kombinierte Inhibition der Dipeptidylpetidase IV und der Aminopeptidase N konnten bereits erste Hinweise auf eine positive Modulation der Inflammation in Reaktion auf Neurodegeneration erhalten werden. Nach einer fokalen Ischämie im Tiermodel verringerte sich durch kombinierte DP IV-/APN-Inhibition das Infarktvolumen (bislang unveröffentlichte Ergebnisse). In hippokampalen organotypischen Schnittkulturen kann durch Inflammation eine Degeneration des Gewebes, wobei auch die Neurone untergehen, ausgelöst werden. Diese Degeneration hängt von aktivierter Mikroglia ab, die bei inflammatorischen Prozessen im Gehirn eine entscheidende Rolle spielt. Auch hier konnte die Degeneration des Nervengewebes durch eine kombinierte Inhibition von DP IV/APN verringert werden, was auf eine Eindämmung/Modulation der Inflammation schließen lässt. Der genaue Mechanismus dieser Protektion ist aber noch nicht verstanden und wird zur Zeit genauer untersucht. Nichtsdestotrotz kann aber bereits festgestellt werden, dass deutliche Anzeichen für eine Modulation der Inflammationsreaktion in einer Art und Weise vorhanden sind, die eine fortschreitende, sich selbst perpetuierende Degeneration von Nervengewebe unterbricht. Damit bleiben degenerative Prozesse auf die primären Ursachen beschränkt; eine Beschleunigung/Ausbreitung der Degeneration durch Inflammation wird verhindert. Diese deutliche Verlangsamung der Pathogenese könnte auch beim Patienten zu einer deutlichen Verbesserung der Symptomatik vor allem in frühen bis mittleren Stadien der Erkrankung führen, bzw. die späteren Stadien, die durch vollständige Pflegeabhängigkeit charakterisiert sind, möglicherweise um Jahre verzögern.

Der Einsatz von DP IV- und APN-Inhibitoren würde bei den genannten Erkrankungen vor allem in den frühen Stadien eine gänzlich neuartige, vorraussichtlich sehr effektive, möglicherweise kostengünstige Therapieform und einen wertvollen alternativen Bestandteil der bestehenden Therapiekonzepte darstellen.

Die vorliegende Erfindung betrifft die Verwendung von Inhibitoren der Dipeptidylpeptidase IV (DP IV) oder von Inhibitoren von Enzymen mit gleicher Substratspezifität (DP IV-analoger Enzymaktivität) in Kombination mit Inhibitoren der Alanyl-Aminopeptidase (Aminopeptidase N, APN) oder von Inhibitoren von Enzymen mit gleicher Substratspezifität (APN-analoger Enzymaktivität) zur Herstellung eines Arzneimittels zur Reduktion neurodegenerativer Läsionen, die durch die Bildung von Aggregaten mißgefalteter Proteine (wie NFT) ausgelöst werden, und/oder zur Eindämmung/Modulation von Inflammation, die diese Läsionen beschleunigt.

Entsprechend der erfindungsgemäßen Verwendung sind die Inhibitoren der DP IV oder von Enzymen mit gleilcher Substratspezfität oder DP IV-analoger Enzymaktivität bevorzugt Xaa-Pro-Dipeptide (Xaa = α-Aminosäure bzw. seitenkettengeschütztes Derivat), entsprechende Derivate, vorzugsweise Dipeptidphosphonsäurediarylester, Dipeptidboronsäuren (z.B. Pro-boro-Pro) und deren Salze, Xaa-Xaa-(Trp)-Pro-(Xaa)n-Peptide (Xaa = α-Aminosäure, n = 0 bis 10), entsprechende Derivate und deren Salze, Aminosäure(Xaa)-amide, entsprechende Derivate und deren Salze, wobei Xaa eine α-Aminosäure bzw. ein seitenkettengeschütztes Derivat, vorzugsweise N^{ε}-4-Nitrobenzyloxycarbonyl-L-Lysin, L-Isoleucin, L-Valin, L-Tryptophan, L-Prolin, ist und als Amidstruktur cyclische Amine, z.B. Pyrrolidin, Piperidin, Thiazolidin und deren Derivate fungieren, und/oder Tryptophan-1,2,3,4-tetrahydroisochinolin-3-carbonsäure-derivate (TSL) und (2S,2S',2S")-2-[2'-[2"-amino-3"-(indol-3"'-yl)-1"-oxoprolyl]-1',2',3',4'-tetrahydro-6'8'-dihydroxy-7-methoxyisochinol-3-yl-carbonyl-amino]-4-hydromethyl-5-hydropentansäure (TMC-2A).

Gemäß einer bevorzugten Verwendung werden als Inhibitoren oder DP IV oder von Enzymen mit gleicher Substratspezifität oder DP IV-analoger Enzymaktivität Aminosäureamide, bevorzugt N^{ε}-4-Nitrobenzyloxycarbonyl-L-Lysin-thiazolidid, pyrrolidid und -piperidid sowie das entsprechende 2-Cyanothiazolidid-, 2-Cyanopyrrolidid- und 2-Cyanopiperididderivat, als DP IV-Inhibitoren eingesetzt.

Entsprechend der erfindugnsgemäßen Verwendung werden als Inhibitoren der APN oder von Enzymen mit gleicher Substratspezifität oder APN-analoger Enzymaktivität bevorzugt Actinonin, Leuhistin, Phebestin, Amastatin, Bestatin, Probestin, β-Aminothiole, α-Aminophosphinsäuren, α-Aminophosphinsäurederivate, vorzugsweise D-ψ-Phe-PO(OH)-CH₂]-Phe-Phe, und deren Salze eingesetzt.

Entsprechend der erfindungsgemäßen Verwendung werden die Inhibitorkombinationen gemäß der vorstehenden Angaben eingesetzt zur Vorbeugung und Thera-pie von Morbus Alzheimer, der Pick'schen Erkrankung, der Progressiven Supranukleären Palsy, der kortikobasalen Degeneration, der frontotemporaten Demenz, von Morbus Parkinson, insbesondere Parkinsonismus gekoppelt an das Chromosom 17, von Morbus Huntington und von durch Prionen bedingten Krankheitszuständen.

Die Inhibitorkombinationen werden simultan mit an sich bekannten Träger-, Hilfs- und/oder Zusatzstoffen verabreicht. Die Verabreichung erfolgt einerseits als systemische Anwendung zur oralen, transdermalen, percutanen, intravenösen, subcutanen, intracutanen, intramuskulären, rektalen, vaginalen und sublingualen Applikation und andererseits als topische Anwendung in Form von Cremes, Salben, Pasten, Gelen, Lösungen, Sprays, Liposomen bzw. Nanosomen, "pegylierten" Formulierungen, degradierbaren Depot-Matrices, Schüttelmixturen, Hydrokolloidverbänden, Pflastern, Mikroschwämmen, Prepolyomeren und ähnlichen neuen Trägersubstraten, Jet-Injektionen bzw. anderen dermatologischen Grundlagen/Vehikeln, einschließlich instillativer Applikation.

Alternativ dazu werden die Inhibitorkombinationen in der Art verwendet, bei der die Inhibitoren der Dipeptidylpeptidase IV (DP IV) oder die Inhibitoren von Enzymen mit gleicher Substratspezifität (DP IV-analoger Enzymaktivität) und die Inhibitoren der Alanyl-Aminopeptidase (Aminopeptidase N, APN) oder die Inhibitoren von Enzymen mit gleicher Substratspezifität (APN-analoger Enzymaktivität) in räumlich getrennter Formulierung in Kombination mit an sich bekannten Träger-, Hilfs- und/oder Zusatzstoffen gleichzeitig oder zeitlich unmittelbar aufeinanderfolgend mit dem Ziel einer gemeinsamen Wirkung verabreicht werden.

Die erfindungsgemäß applizierten Inhibitoren der Dipeptidylpeptidase IV oder Inhibitoren von Enzymen mit gleicher Substratspezifität (DP IV-analoger Enzymaktivität) und Inhibitoren der Aminopeptidase N oder Inhibitoren von Enzymen gleicher Substratspezifität (APN-analoger Enzymaktivität) können in pharmazeutisch anwendbaren Formulierungskomplexen als Inhibitoren, Substrate, Pseudosubstrate, inhibitorisch wirkende Peptide und Peptidderivate sowie als Antikörper dieser Enzyme zur Anwendung kommen. Die Inhibitoren gemäß der Erfindung werden in Kombination von mehreren von innen, vorzugsweise in Kombination von zweien von ihnen, eingesetzt.

Bevorzugte Effektoren sind für die DP IV Xaa-Pro-Dipeptide, entsprechende Derivate, vorzugsweise Dipeptidphosphonsäurediarylester, Dipeptidboronsäuren (z. B. Pro-boro-Pro) und deren Salze, Xaa-Xaa-(Trp)-Pro-(Xaa)n-Peptide (n = 0 bis 10), entsprechende Derivate und deren Salze bzw. Aminosäure (Xaa)-amide, entsprechende Derivate und deren Salze, wobei Xaa eine α-Aminosäure/Iminosäure bzw. ein α-Aminosäurederivatj/Iminosäurederivat, vorzugsweise N^{ε}-4-Nitrobenzyl-oxycarbonyl-L-Lysin, L-Prolin, L-Tryptophan, L-Isoleucin, L-Valin ist und als Amidstruktur cyclische Amine, z.B. Pyrrolidin, Piperidin, Thiazolidin und deren Derivate fungieren. Derartige Verbindungen und deren Herstellung wurden in einem früheren Patent beschrieben (K. Neubert et al. DD296075A5). Weiter können als Effektoren für die DP IV mit Vorteil Tryptophan-1,2,3,4-tetrahydroisochinolin-3-carbonsäurederivate (TSL) und (2S,2S',2S")-2-[2'-[2"-amino-3"-(indol-3"'-yl)-1"-oxoprolyl]-1',2',3',4'-tetrahydro-6'8'dihydroxy-7-methoxyisochinol-3-yl-carbonyl-amino]-4-hydromethyl-5-hydropentansäure (TMC-2A) verwendet werden. Ein beispielhafter, mit Vorteil verwendbarer Inhibitor von DP IV ist Lys[Z(NO₂]-thiazolidid, worin Lys für einen L-Lysin-Rest steht und Z(NO₂) für 4-Nitrobenzyloxycarbonyl steht (vgl. DD-A 296075).

Als Inhibitoren der Alanyl/aminopeptidase (Aminopeptidase N, APN) kommen erfindungsgemäß beispielsweise Actinonin, Leuhistin, Phebestin, Amastatin, Bestatin, Probestin, β-Aminothiole, α-Aminophosphinsäuren, α-Aminophosphinsäurederivate, vorzugsweise D-Phe-ψ-PO(OH)-CH₂]-Phe-Phe und deren Salze in Betracht. Bevorzugte Inhibitoren für die Alanyl-Aminopeptidase sind Bestatin (Ubenimex), Actinonin, Probestin, Phebestin, RB3014 oder Leuhistin.

Die Inhibitoren oder diese enthaltende pharmazeutische Zubereitungen werden simultan mit bekannten Trägerstoffen verabreicht. Von der Erfindung umfaßt ist auch die verabreichung pharmazeutischer Zubereitungen, die zwei oder mehrere der Inhibitoren der DP IV oder Inhibitoren von Enzymen mit DP IV-analoger Enzymaktivität und der APN oder Inhibitoren von Enzymen mit APN-analoger Enzymaktivität in räumlich getrennter Formulierung in Kombination mit an sich bekannten Träger-, Hilfs- und/oder Zusatzstoffen umfassen zur gleichzeitigen oder zeitlich unmittelbar aufeinanderfolgenden Verabreichung mit dem Ziel einer gemeinsamen Wirkung.

Die Verabreichung erfolgt einerseits als topische Applikation in Form von z.B. Cremes, Salben, Pasten, Gelen, Lösungen, Sprays, Liposomen und Nanosomen, Schüttelmixturen, "pegylierten" Formulierungen, degradierbaren (d. h. unter physiologischen Bedingungen abbaubaren) Depot-Matrices, Hydrokolloidverbänden, Pflastern, Mikroschwämmen, Prepolymeren und ähnlichen neuen Trägersubstraten, Jet-Injektion bzw. anderen dermatologischen Grundlagen/Vehikeln einschließlich instillativer Applikation, und andererseits als systemische Applikation zur oralen, transdermalen, intravenösen, subcutanen, intracutanen, intramuskulären Anwendung in geeigneten Rezepturen bzw. in geeigneter Galenik.

Erfindungsgemäß kommen die Inhibitoren sowie Zubereitungen daraus, die mehrere von den genannten Inhibitoren und gegebenenfalls noch weitere Komponenten wie weitere Inhibitoren, sowie pharmazeutisch verträgliche Zusatz-, Hilfs- oder Trägerstoffe enthalten, bei einer ganzen Anzahl von Demenzerkrankungen bzw. Zuständen mit Bildung neurofibrillärer Tangles (NFT), sogenannten Tauopathien, und anderen Erkrankungen mit abnormalen Proteinakkumulationen vorbeugend oder therapierend zur Anwendung.

Die Erfindung betrifft auch die Verwendung zur Therapie und Prävention von neurodegenerativen Erkrankungen, deren Pathogenese inflammationsabhängig verläuft (siehe oben). So können beispielhaft genannt werden: Morbus Alzheimer, Morbus Parkinson, Morbus Huntington, die von Prionen verursachten Erkrankungen und weitere Tauopathien wie die Pick'sche Erkrankung, die progressive supranukleäre Palsy, die kortikobasale Degeneration und die frontotemporalen Demenz. Dabei umfaßt die Verwendung die Verabreichung von Inhibitoren der Dipeptidylpeptidase IV (DP IV) oder von Inhibitoren von Enzymen mit gleicher Substratspezifität (DP IV-analoger Enzymaktivität) und von Inhibitoren der Alanyl-Aminopeptidase (Aminopeptidase N, APN) oder von Inhibitoren von Enzymen gleicher Substratspezifität (APN-analoger Enzymaktivität) an einen Patienten, der zur Prävention und/oder Therapie der obengenannten Erkrankungen oder Zustände einer Behandlung bedarf.

In besonders bevorzugten Ausführungsformen der Erfindung werden an einen unter einer oder mehreren der nachfolgend im einzelnen genannten Erkrankungen oder Zustände leidenden oder einer Prävention der nachfolgend genannten Krankheiten oder Zustände bedürfenden Patienten mehrere Inhibitoren der genannten Enzyme oder eine oder mehrere, diese Inhibitoren in Kombination enthaltende pharmazeutische Zubereitung(en) verabreicht, die gewählt sind aus Inhibitoren der DP IV und besonders bevorzugt aus Xaa-Pro-Dipeptiden (Xaa = α-Aminosäure bzw. seitenkettengeschütztes Derivat), entsprechenden Derivaten, vorzugsweise Dipeptidphosphonsäurediarylestem, Dipeptidboronsäuren (z.B. Pro-boro-Pro) und deren Salzen, Xaa-Xaa-(Trp)-Pro-(Xaa)n-Peptiden (Xaa = α-Aminosäure, n = 0 bis 10), entsprechenden Derivaten und deren Salzen, Aminosäure (Xaa)-amiden, entsprechende Derivaten und deren Salzen, wobei Xaa eine α-Aminosäure bzw. ein seitenkettengeschütztes Derivat, vorzugsweise N^{ε}-4-Nitrobenzyloxycartionyl-L-Lysin, L-Isoleucin, L-Valin, L-Tryptophan, L-Prolin, ist und als Amidstruktur cyclische Amine, z.B. Pyrrolidin, Piperidin, Thiazolidin und deren Derivate fungieren, und/oder Tryptophan-1,2,3,4-tetrahydroisochinolin-3-carbonsäure-derivate (TSL) und (25,25',25")-2-[2'-[2"-amino-3"-(indol-3"'-y1)-1"-oxoprolyl]-1',2',3',4'-tetrahydro-6'8'-dihydroxy-7-methoxyisochinol-3-yl-carbonyl-amino]-4-hydromethyl-5-hydropentansäure (TMC-2A), und Inhibitoren der APN, besonders bevorzugt Actinonin, Leuhistin, Phebestin, Amastatin, Bestatin, Probestin, β-Aminothiolen, α-Aminophosphinsäuren, α-Aminophosphinsäurederivaten, vorzugsweise D-ψ-Phe-PO(OH)-CH₂]-Phe-Phe, und deren Salzen.

Gemäß der Erfindung kommen die Inhibitor kombinationen und diese enthaltende pharmazeutische Zubereitungen zur Anwendung bei der Prävention und Therapie von Erkrankungen und bzw. Zuständen mit Bildung neurofibrillärer Tangles (NFT) und anderer abnormaler Proteinakkumulationen. Beispielhaft können genannt werden eine Vorbeugung und Therapie von Morbus Alzheimer, der Pick'schen Erkrankung, der progressiven supranukleären Palsy, der kortikobasalen Degeneration, der frontotemporalen Demenz, von Morbus Parkinson, Morbus Huntington und von durch Prionen bedingten Erkankungen

In erfindungsgemäß besonders bevorzugten Präventions- und/oder Therapieverfahren werden die genannten Inhibitoren der DP IV und APN in der Weise zur Anwendung gebracht, daß zwei oder mehrere der Inhibitoren der DP IV oder Inhibitoren von Enzymen mit DP IV-analoger Enzymaktivität und Inhibitoren der APN oder Inhibitoren von Enzymen mit APN-analoger Enzymaktivität in räumlich getrennter Formulierung in Kombination mit an sich bekannten Träger-, Hilfs- und/oder Zusatzstoffen gleichzeitig oder zeitlich unmittelbar aufeinander folgend mit dem Ziel einer gemeinsamen Wirkung verabreicht werden. Die Verabreichung erfolgt als systemische Anwendung zur oralen, transdermalen, percutanen, intravenösen, subcutanen, intracutanen, intramuskulären, rektalen, vaginalen, sublingualen Applikation zusammen mit an sich bekannten Träger-, Hilfs- und/oder Zusatzstoffen und/oder als topische Anwendung in Form von Cremes, Salben, Pasten, Gelen, Lösungen, Sprays, Liposomen bzw. Nanosomen, "pegylierten" Formulierungen, degradierbaren Depot-Matrices, Schüttelmixturen, Hydrokolloidverbänden, Pflastern, Mikroschwämmen, Prepolyomeren und ähnlichen neuen Trägersubstraten, Jet-Injektionen bzw. anderen dermatologischen Grundlagen/Vehikeln, einschließlich instillativer Applikation.

Die Erfindung wird anhand der nachfolgenden Beispiele näher erläutert. Die Beispiele zeigen bevorzugte Ausführungsformen der Erfindung. Die Erfindung ist jedoch nicht auf die bevorzugten Ausführungsformen beschränkt.

### Beispiele

Transgene Mäuse, die das humane Mikrotubuli-assoziierte Protein Tau mit der Mutation P301L unter der Kontrolle des Maus Prion-Promotors exprimieren, entwickeln altersabhängig neurofibrilläre Tangles (NFT), Neurodegeneration und Defizite im Bewegungsapparat verbunden mit Verhaltensbeeinträchtigungen (Lewis J, McGowan E, Rockwood J, Melrose H, Nacharaju P, Van Slegtenhorst M, Gwinn-Hardy K, Paul Murphy M, Baker M, Yu X, Duff K, Hardy J, Corral A, Lin WL, Yen SH, Dickson DW, Davies P, Hutton M. Neurofibrillary tangles, amyotrophy and progressive motor disturbance in mice expressing mutant (P301 L) tau protein. Nat Genet. 2000, 25(4), 402 - 405). Sie können deshalb als ein vollständiges Modell für die Pathogenese menschlicher Erkrankungen mit NFT, den sogenannten Tauopathien, angesehen werden.

Die ursprüngliche JNPL3 Mauslinie wurden mit B6D2F1 Hybriden gekreuzt erworben (Taconic; Germantown; USA) und die hemizygote F1 entwickelte nach ca. 6 Monaten erste Symptome von sich entwickelnden Bewegungs- und Verhaltenstörungen. Die betroffenen Mäuse waren nicht mehr in der Lage, bei Aufnahme am Schwanz als Fluchtreaktion die Extremitäten zu spreizen, sondern zeigten im Gegenteil spontane Verkrampfungen der Hinterbeine. Nach 10 Monaten zeigten über 90% der Tau-transgenen Mäuse Schwierigkeiten beim Aufrichten und in Hängetests konnte eine angebotene Leine nur noch mit Mühe ergriffen werden, so dass ein Anklammern häufig misslang.

Auf zellulärer Ebene korrelierten damit neuropathologische Veränderungen, deren auffälligste die Bildung von NFT unter anderem im Rückenmark waren. Diese konnten durch Congorot und Thioflavin-S Floureszensmikroskopie sowie Gallyas Silberfärbungen nachgewiesen werden. Darüber hinaus konnte eine somatodendritische, "pre-tangle"-Tau Akkumulation in weiteren Hirnregionen, speziell im Entorhinalen Kortex sowie im Hippokampus nachgewiesen werden.

Um die NFT-Bildung auch in diesen Bereichen zu beschleunigen wurden 6 bis 7 Monate alten transgenen Mäusen fibrilläres Abeta 1-42 in den entorhinalen Kortex oder den Hippokampus stereotaktisch injiziert. Dieses sorgte, je nach Injektionsstelle, für die robuste Ausbildung von NFT-Pathologie im Hippokampus bzw. der Amygdala von transgenen, nicht aber Wildtyp-Kontrolltieren, innerhalb von 2 bis 3 Wochen.

Während dieser Zeit wurde den Tieren alle 2 bis 3 Tage eine Kombination von Inhibitoren gegen DPP IV (I-49) und APN (Bestatinhydrochlorid; Actinonin) in einer Konzentration von 5 bis10 mg/kg Körpergewicht durch i.p. Injektionen verabreicht. Darüber hinaus wurden beide Inhibitoren jeweils auch einzeln bis zu einer Konzentration von 50 mg/kg Körpergewicht getestet.

Durch die stereotaktische Injektion von fibrillärem Abeta in den entorhinalen Kortex der einen Hemisphere und den Hippocampus der kontralateralen Hemisphere kann davon ausgegangen werden, dass Amyloid-Fibrillen noch mindestens 45 Tage nach der Injektion im Gewebe vorhanden sind (Götz J, Chen F, van Dorpe J, Nitsch RM. Formation of neurofibrillary tangles in P301L tau transgenic mice induced by Abeta 42 fibrils. Science 2001, 293(5534), 1491-5.). NFT bildeten sich jeweils in Zellkörpern, deren Axone zu den Injektionsstellen projizierten, also dem Hippokampus bzw. der Amygdala.

Die Anzahl der NFT konnte durch die Gabe sowohl von Inhibitoren der DPP IV als auch von Inhibitoren der APN in beiden Hirnarealen signifikant gesenkt werden. Durch die Kombination beider Inhibitoren ergab sich darüber hinaus eine bessere NFT-Protektion als für jeden einzelnen Inhibitor beobachtet, was für eine über der Addition der Effekte liegende Wirkung der Kombination der einzelnen Inhibitoren spricht.

Gallyas-positive NFT wurden wie in Götz et al. (a. a. O.) beschrieben ausgewertet. Dazu wurden serielle frontale Schnitte durch das Hirn angefertigt und jeder 5. Schnitt in der Nähe der Injektionsstelle wurde nach Gallyas-Färbung analysiert. Gallyas-positve NFT wurden auf 20 standardisierten Schnitten gezählt und aufaddiert. Mittelwert und Standardabweichung wurden für jeweils drei Mäuse ermittelt.

Darüber hinaus ergab sich auch eine Verbesserung des allgemeinen Zustandes der Versuchstiere. So setzten die Defizite des Bewegungsapparates im Beobachtungszeitraum nicht oder nur so abgeschwächt ein, dass die normale Fluchtreaktion durch Extension der Hinterbeine kaum beeinträchtigt wurde. Auch eine zusammengekauerte Haltung mit verkrampften Pfoten als Sitzposition wurde nur selten beobachtet. Auch nicht indirekt mit dem Bewegungsapparat zusammenhängende Parameter konnten als verbessert angesehen werden. Mit DPP IV-/APN-Inhibitoren behandelte Tiere verloren nicht an Gewicht und zeigten kaum beeinträchtigtes Putzverhalten.

Zusammenfassend konnte also eine gute Korrelation im Rückgang der Neuropathologie, im Besonderen der NFT-Bildung, mit einer Verbesserung des Zustandes der Mäuse beobachtet werden. Das Ausbleiben der Verhaltensdefizite hing jedoch vermutlich nicht oder nur indirekt mit der Reduktion der NFT-Bildung in den analysierten Hirnregionen zusammen. Eine Korrelation der Verhaltensabnormalitäten mit der bereits beschriebenen NFT-Pathologie und damit einhergehenden Neurodegeneration, insbesondere im Rückenmark und im Hirnstammbereich (Lewis et al., a. a. O.) kann jedoch als sehr wahrscheinlich angesehen werden. Somit wären auch die Verbesserungen in der Verhaltenssymptomatik entweder auf eine Protektion der Nervenzellen durch die getestete Wirkstoffkombination oder zumindest eine Verlangsamung der Pathogenese zurückzuführen.

## Patentansprüche

1. Verwendung von Inhibitoren der Dipeptidylpeptidase IV (DP IV) oder von Inhibitoren von Enzymen mit gleicher Substratspezifität (DP IV-analoger Enzymaktivität) in Kombination mit Inhibitoren der Alanyl-Aminopeptidase (Aminopeptidase N, APN) oder von Inhibitoren von Enzymen mit gleicher Substratspezifität (APN-analoger Enzymaktivität) zur Herstellung eines Medikaments zur Vorbeugung und Therapie von Morbus Alzheimer, der Pick'schen Erkrankung, der progressiven supranukleären Palsy, der kortikobasalen Degeneration, der frontotemporalen Demenz, von Morbus Parkinson, insbesondere Demenz mit Parkinsonismus gekoppelt an das Chromosom 17, von Morbus Huntington und von durch Prionen bedingten Krankheitszuständen, spongiformer Enzephalopathie sowie zur Therapie und Prävention von Demenzerkrankungen oder -zuständen durch Reduktion neurodegenerativer Läsionen, die durch die Bildung von Aggregaten mißgefalteter Proteine ausgelöst werden, und/oder durch Eindämmung/Modulation von Inflammation, die diese Läsionen beschleunigt.

2. Verwendung nach Anspruch 1, worin die Inhibitoren der DP IV ausgewählt sind aus der Gruppe, die besteht aus
- Xaa-Pro-Dipeptiden, entsprechenden Derivaten oder deren Salzen, worin Xaa eine α-Aminosäure oder ein seitenkettengeschütztes Derivat ist;
- Xaa-Xaa-(Trp)-Pro-(Xaa)n-Peptiden, entsprechenden Derivaten oder deren Salzen,
worin Xaa eine α-Aminosäure ist und n für eine ganze Zahl von 0 bis 10 steht;
- Aminosäure(Xaa)-amiden, entsprechenden Derivaten oder deren Salzen,
worin Xaa eine α-Aminosäure oder ein seitenkettengeschütztes Derivat ist und als Amidstruktur cyclische Amine fungieren;
- Tryptophan-1,2,3,4-tetrahydroisochinolin-3-carbonsäure-Derivate (TSL); und
- (25,25',25")-2-[2'-[2"-Amino-3"-(indol-3"-yl)-1"-oxoprolyl]-1',2',3',4'-tetrahydro-6'8'-dihydroxy-7-methoxyisochinol-3-yl-carbonylamino]-4-hydromethyl-5-hydropentansäure (TMC-2A).

3. Verwendung nach Anspruch 2, worin das Xaa-Pro-Dipeptid ausgewählt ist aus der Gruppe, die besteht aus Dipeptidphosphonsäurediarylestern und Dipeptidboronsäuren.

4. Verwendung nach Anspruch 3, worin die Dipeptidboronsäure Pro-Boro-Pro ist.

5. Verwendung nach Anspruch 2, worin das Xaa des Aminosäure(Xaa)-amids ausgewählt ist aus der Gruppe, die besteht aus N^{ε}-4-Nitrobenzyloxycarbonyl-L-Lysin, L-Prolin, L-Tryptophan, L-Isoleucin und L-Valin.

6. Verwendung nach Anspruch 2, worin das cyclische Amin ausgewählt ist aus der Gruppe, die besteht aus Pyrrolidin, Piperidin, Thiazolidin und deren Derivaten.

7. Verwendung nach einem der Ansprüche 2 bis 6, worin das Aminosäure(Xaa)-amid ausgewählt ist aus der Gruppe, die besteht aus N^{ε}-4-Nitrobenzyloxy-carbonyl-L-Lysin-thiazolidid, N^{ε}-4-Nitrobenzyloxy-carbonyl-L-Lysin-pyrrolidid, N^{ε}-4-Nitrobenzyloxy-carbonyl-L-Lysin-piperidid, N^{ε}-4-Nitrobenzyloxy-carbonyl-L-Lysin-2-cyanothiazolidid, N^{ε}-4-Nitrobenzyloxycarbonyl-L-Lysin-2-cyanopyrrolidid und N^{ε}-4-Nitrobenzyloxy-carbonyl-L-Lysin-2-cyanopiperidid.

8. Verwendung nach einem der Ansprüche 2 bis 7, worin der DP IV-Inhibitor Lys[Z(NO₂)]-thiazolidid, worin Lys für einen L-Lysin-Rest und Z(NO₂) für 4-Nitrobenzyloxycarbonyl steht, ist.

9. Verwendung nach einem der Ansprüche 1 bis 8, worin die Inhibitoren der APN ausgewählt sind aus der Gruppe, die besteht aus Actinonin, Leuhistin, Phebestin, Amastatin, Bestatin, RB3014, Probestin, β-Aminothiolen, α-Aminophosphinsäuren, α-Aminophosphinsäurederivate, vorzugsweise D-Phe-ψ-[PO(OH)-CH₂]-Phe-Phe und deren Salzen.

10. Verwendung einer pharmazeutischen Zubereitung, umfassend Inhibitorkombinationen nach einem der Ansprüche 1 bis 9, in Kombination mit an sich bekannten Träger-, Hilfs- und/oder Zusatzstoffen.

11. Verwendung mehrerer pharmazeutischen Zubereitungen, umfassend einzelne Inhibitoren oder Inhibitorkombinationen nach einem der Ansprüche 1 bis 9, in Kombination mit an sich bekannten Träger-, Hilfs- und/oder Zusatzstoffen.

12. Verwendung nach einem der Ansprüche 1 bis 11, worin zwei oder mehrere der Inhibitoren der DP IV oder der Inhibitoren von Enzymen mit DP IV-analoger Enzymaktivität und der Inhibitoren der APN oder der Inhibitoren von Enzymen mit APN-analoger Enzymaktivität in räumlich getrennter Formulierung gleichzeitig oder zeitlich unmittelbar aufeinanderfolgend mit dem Ziel einer gemeinsamen Wirkung verabreicht werden.

13. Verwendung nach einem der Ansprüche 1 bis 12 für die systemische Anwendung zur oralen, transdermalen, intravenösen, percutanen, subcutanen, intracutanen, intramuskulären, rektalen, vaginalen oder sublingualen Applikation zusammen mit an sich bekannten Träger-, Hilfs- und/oder Zusatzstoffen.

14. Verwendung nach einem der Ansprüche 1 bis 12 für die topische Anwendung in Form von Cremes, Salben, Pasten, Gelen, Lösungen, Sprays, Liposomen bzw. Nanosomen, "pegylierten" Formulierungen, degradierbaren Depot-Matrices, Schüttelmixturen, Hydrokolloidverbänden, Pflastern, Mikroschwämmen, Prepolymeren und ähnlichen neuen Trägersubstraten, Jet-Injektionen bzw. anderen dermatologischen Grundlagen/Vehikeln, einschließlich instillativer Applikation.

## Claims

1. Use of inhibitors of dipeptidylpeptidase IV (DP IV) or of inhibitors of enzymes having a similar substrate specificity (DP IV-analogous enzyme activity) in combination with inhibitors of alanyl aminopeptidase (aminopeptidase N, APN) or of inhibitors of enzymes having a similar substrate specificity (APN-analogous enzyme activity) for the preparation of a medicament for a prevention and therapy of Morbus Alzheimer, of Morbus Pick, of the progressive supranuclear palsy, of the corticobasal degeneration, of the frontotemporal dementia, of Morbus Parkinson, particularly of dementia in combination with Morbus Parkinson, coupled to chromosome 17, of Morbus Huntington and of disease conditions caused by priones, spongiformer enzephalopathia as well as for a therapy and prevention of dementia diseases or conditions by the reduction of neurodegenerative lesions, caused by the formation of aggregates of misfold proteins and/or by the control/modulation of inflammation accelerating such lesions.

2. The use according to claim 1, wherein the inhibitors of DP IV are selected from the group, consisting of
- Xaa-Pro-dipeptides, corresponding derivatives or salts thereof,
wherein Xaa is an α-amino acid or a side-chain protected derivative;
- Xaa-Xaa-(Trp)-Pro-(Xaa)n peptides, corresponding derivatives or salts thereof,
wherein Xaa is an α-amino acid and n is an integer from 0 to 10;
- amino acid (Xaa) amides, corresponding derivatives or salts thereof,
wherein Xaa is an α-amino acid or a side-chain protected derivative and cyclic amines act as the amide structure;
- tryptophane-1,2,3,4-tetrahydroisochinoline-3-carboxylic acid derivatives (TSL); and
- (2S,2S',2S")-2-[2'-[2"-amino-3"-(indol-3"'-yl)-1"-oxoprolyl]-1',2',3',4'-tetrahydro-6'8'-dihydroxy-7-methoxyisochinol-3-yl-carbonyl-amino]-4-hydromethyl-5-hydropentanoic acid (TMC-2A).

3. The use according to claim 2, wherein the Xaa-Pro-dipeptide is selected from the group consisting of dipeptide phosphonic acid diaryl esters and dipeptide boronic acids.

4. The use according to claim 3, wherein the dipeptide boronic acid is Pro-boro-Pro.

5. The use according to claim 2, wherein the Xaa of the amino acid (Xaa) amide is selected from the group consisting of N^{ε}-4-nitrobenzyl oxycarbonyl-L-lysine, L-proline, L-tryptophane, L-isoleucine and L-valine.

6. The use acccording to claim 2, werein the cyclic amine is selected from the group consisting of pyrrolidine, piperidine, thiazolidine and derivatives thereof.

7. The use according to any of claims 2 to 6, wherein the amino acid (Xaa) amide is selected from the group consisting of N^{ε}-4-nitrobenzyloxy carbonyl-L-lysine thiazolidid, N^{ε}-4-nitrobenzyloxy carbonyl-L-lysine pyrrolidid, N^{ε}-4-nitrobenzyloxy carbonyl-L-lysine piperidid, N^{ε}-4-nitrobenzyloxy carbonyl-L-lysine-2-cyano thiazolidid, N^{ε}-4-nitrobenzyloxy carbonyl-L-lysine-2-cyano pyrrolidid and N^{ε}-4-nitrobenzyloxy carbonyl-L-lysine-2-cyano piperidid.

8. The use according to any of claims 2 to 7, wherein the inhibitor of DP IV is Lys[Z(NO₂)]-thiazolidid, worin Lys is a L-Lysin residue and Z(NO₂) is 4-nitrobenzyloxy carbonyl.

9. The use according to any of claims 1 to 8, wherein the inhibitors of APN are selected from the group consisting of actinonin, leuhistin, phebestin, amastatin, bestatin, RB3014, probestin, β-aminothiols, α-aminophosphinic acids, α-amino phosphinic acid derivatives, preferably D-Phe-ψ-[PO(OH)-CH₂]-Phe-Phe, and salts thereof.

10. Use of a pharmaceutical composition comprising the inhibitor combinations according to any of claims 1 to 9, in combination with per se known carrier substances, additives and/or auxiliary substances.

11. Use of several pharmaceutical preparations comprising single inhibitors or inhibitor combinations according to any of claims 1 to 9, in combination with per se known carrier substances, additives and/or auxiliary substances.

12. The use according to any of claims 1 to 11, wherein two or more of the inhibitors of the DP IV or of the inhibitors of enzymes having DP IV-analogous enzyme activity and of the inhibitors of APN or of the inhibitors of enzymes having APN-analogous enzyme activity are administered in a spaced-apart formulation simultaneously or, with respect to time, immediately successively with the aim of a joint effect.

13. The use according to any of claims 1 to 12 for a systemic administration for an oral, transdermal, intravenous, percutaneous, subcutaneous, intracutaneous, intramuscular, rectal, vaginal or sublingual application, together with per se known carrier substances, auxiliary substances and/or additives.

14. The use according to any of claims 1 to 12 for a topical administration in the form of cremes, ointments, pastes, gels, solutions, sprays, liposomes or nanosomes, "pegylated" formulations, degradable depot matrices, agitated mixtures (lotions), hydrocolloid dressings, plasters, microsponges, prepolymers and similar novel carrier substrates, jet injections, and other dermatological bases/vehicles including instillative application.

## Revendications

1. Utilisation d'inhibiteurs de la dipeptidylpeptidase IV (DP IV) ou d'inhibiteurs d'enzymes possédant une spécificité de substrat identique (activité enzymatique analogue à celle de la DP IV) en combinaison avec des inhibiteurs de l'alanyl-aminopeptidase (aminopeptidase N, APN) ou d'inhibiteurs d'enzymes possédant une spécificité de substrat identique (activité enzymatique analogue à celle de la APN) pour la préparation d'un médicament destiné à la prévention et à la thérapie de la maladie d'Alzheimer, de la maladie de Pick, de la paralysie supranucléaire progressive, de la dégénérescence cortico-basale, de la démence frontotemporale, de la maladie de Parkinson, en particulier de la démence parkinsonienne couplée au chromosome 17, de la maladie de Huntington et des états de maladies à prions, de l'encéphalopathie spongiforme, et pour la thérapie et la prévention de maladies démentielles ou d'états de démence par réduction des lésions neurodégénératives qui sont déclenchées par la formation d'agrégats de protéines présentant un repliement erroné et/ou par enrayement/modulation de l'inflammation qui accélère ces lésions.

2. Utilisation selon la revendication 1, dans laquelle les inhibiteurs de la DP IV sont choisis parmi le groupe qui est constitué par :
- des dipeptides Xaa-Pro, des dérivés correspondants ou leurs sels, Xaa représentant un acide α aminé ou un dérivé dont la chaîne latérale est protégée ;
- des peptides Xaa-Xaa-(Trp)-Pro (Xaa)n, des dérivés correspondants ou leurs sels, Xaa représentant un acide α aminé et n représentant un nombre entier de 0 à 10 ;
- des amides d'acides aminés (Xaa), des dérivés correspondants ou leurs sels, Xaa représentant un acide α aminé ou un dérivé dont la chaîne latérale est protégée, et qui font office d'amines cycliques possédant une structure amide ;
- des dérivés de l'acide tryptophane-1,2,3,4-tétrahydroisoquinoléine-3-carboxylique (TSL) ; et
- l'acide (2S,2S',2S")-2-[2'-[2"-amino-3"-(indol-3"'-yl)-1"-oxoprolyl]-1', 2',3',4'-tétrahydro-6',8'-dihydroxy-7-méthoxyisoquinol-3-yl-carbonylamino]-4-hydroxyméthyl-5-hydropentanoïque (TMC-2A).

3. Utilisation selon la revendication 2, dans laquelle le dipeptide Xaa-Pro est choisi parmi le groupe qui est constitué par des esters diaryliques de l'acide dipeptidophosphonique et par des acides dipeptidoboriques.

4. Utilisation selon la revendication 3, dans laquelle l'acide dipeptidoborique est Pro-Boro-Pro.

5. Utilisation selon la revendication 2, dans laquelle le groupe Xaa de l'amide de l'acide aminé (Xaa) est choisi parmi le groupe qui est constitué par la N^{ε}-4-nitrobenzyloxycarbonyl-L-lysine, la L-proline, le L-tryptophane, la L-isoleucine et la L-valine.

6. Utilisation selon la revendication 2, dans laquelle l'amine cyclique est choisie parmi le groupe qui est constitué par la pyrrolidine, la pipéridine, la thiazolidine et leurs dérivés.

7. Utilisation selon l'une quelconque des revendications 2 à 6, dans laquelle l'amide de l'acide aminé (Xaa) est choisi parmi le groupe qui est constitué par le N^{ε}-4-nitrobenzyloxy-carbonyl-L-lysine-thiazolidide, le N^{ε}-4-nitrobenzyloxy-carbonyl-L-lysine-pyrrolidide, le N^{ε}-4-nitrobenzyloxy-carbonyl-L-lysine-pipéridide, le N^{ε}-4-nitrobenzyloxy-carbonyl-L-lysine-2-cyanothiazolidide, le N^{ε}-4-nitrobenzyloxy-carbonyl-L-lysine-2-cyanopyrrolidide et le N^{ε}-4-nitrobenzyloxy-carbonyl-L-lysine-2-cyanopipéridide.

8. Utilisation selon l'une quelconque des revendications 2 à 7, dans laquelle l'inhibiteur de la DP IV est le Lys[Z(NO₂)]-thiazolidide, dans lequel Lys représente un résidu de L-lysine et Z(NO₂) représente un groupe 4-nitrobenzyloxycarbonyle.

9. Utilisation selon l'une quelconque des revendications 1 à 8, dans laquelle les inhibiteurs de la APN sont choisis parmi le groupe qui est constitué par l'actinonine, la leuhistine, la phébestine, l'amastatine, la béstatine, RB3014, la probestine, les β-aminothiols, des acides α-aminophosphiniques, des dérivés de l'acide α-aminophosphinique, de préférence par le groupe D-Phe-ψ[PO(OH)-CH₂]-Phe-Phe et leurs sels.

10. Utilisation d'une préparation pharmaceutique, comprenant des combinaisons d'inhibiteurs selon l'une quelconque des revendications 1 à 9, en combinaison avec des supports, des adjuvants et/ou des additifs connus en soi.

11. Utilisation de plusieurs préparations pharmaceutiques, comprenant des inhibiteurs individuels ou des combinaisons d'inhibiteurs selon l'une quelconque des revendications 1 à 9, en combinaison avec des supports, des adjuvants et/ou des additifs connus en soi.

12. Utilisation selon l'une quelconque des revendications 1 à 11, dans laquelle deux inhibiteurs ou plus parmi des inhibiteurs de la DP IV ou des inhibiteurs d'enzymes possédant une activité enzymatique analogue à celle de la DP IV et des inhibiteurs de la APN ou des inhibiteurs d'enzymes possédant une activité enzymatique analogue à celle de la APN sont administrés dans des formulations séparées dans l'espace, de manière simultanée ou d'une manière telle qu'ils se succèdent directement dans le temps dans le but d'obtenir un effet commun.

13. Utilisation selon l'une quelconque des revendications 1 à 12 pour l'administration systémique à des fins d'application par voie orale, transdermique, intraveineuse, percutanée, sous-cutanée, intracutanée, intramusculaire, rectale, vaginale ou sublinguale, de manière conjointe avec des supports, des adjuvants et/ou des additifs connus en soi.

14. Utilisation selon l'une quelconque des revendications 1 à 12, pour l'application locale sous la forme de crèmes, d'onguents, de pâtes, de gels, de solutions, de sprays, de liposomes respectivement de nanosomes, de formulations « pégylées », de matrices retard dégradables, de mélanges à secouer, de pansements hydrocolloïdaux, d'emplâtres, de microéponges, de prépolymères et de nouveaux substrats de support analogues, d'injections par jets, respectivement d'autres bases/véhicules dermatologiques, y compris une application par instillation.
